# EUROPEAN PATENT SPECIFICATION

(11) **EP 4 197 440 B1**
(45) Date of publication and mention of the grant of the patent: **30.09.2026**
(21) Application number: 22211913.3
(22) Date of filing: 07.12.2022
(51) Int. Cl.: A61B 5/265, A61B 5/266, A61B 5/268, A61B 5/271, A61B 5/308, A61B 5/31, A61B 5/344, A61B 5/369, H05K 1/00, H05K 1/09, H05K 1/03

(54) **SURFACE ELECTRODE FOR PATIENT MONITORING**
OBERFLÄCHENELEKTRODE ZUR PATIENTENÜBERWACHUNG
ÉLECTRODE DE SURFACE POUR SURVEILLANCE DE PATIENT

(30) Priority: 20.12.2021 US 202117556339
(43) Date of publication of application: 21.06.2023
(73) Proprietor: GE Precision Healthcare LLC, Wauwatosa, WI 53226 (US)
(72) Inventor: CRYNOCK, Stephen M., Wauwatosa, 53226 (US); GABEL, Christopher, Wauwatosa, 53226 (US); WANG, Yanju, Wauwatosa, 53226 (US); WALGRAVE, Sean R., Wauwatosa, 53226 (US); SAUER, Dennis A., Wauwatosa, 53226 (US)
(74) Representative: Kilburn & Strode LLP

(56) References cited:
- EP-A2- 2 213 257
- EP-B1- 2 827 768
- WO-A1-00/19892
- WO-A1-2020/214812
- WO-A2-2022/031166
- US-A1- 2008 288 026
- US-A1- 2021 345 899
- HYUN SUK HUN ET AL: "Silver and epoxy binder-based printed electrodes and the effect of silver nanoparticles on stretchability", JOURNAL OF MATERIALS SCIENCE: MATERIALS IN ELECTRONICS, CHAPMAN AND HALL, LONDON, GB, vol. 30, no. 19, 5 September 2019 (2019-09-05), pages 17591 - 17600, XP036904499, ISSN: 0957-4522, [retrieved on 20190905], DOI: 10.1007/S10854-019-02108-Z

## Description

### BACKGROUND

The present disclosure generally relates to surface electrodes and surface electrode sets attachable to a patient's skin for physiological patient monitoring, and specifically to surface electrodes utilizing a wet electrode.

Surface electrodes, which are adhered to patient's skin surface, enable electrical contact between a patient's skin and a conductor. Surface electrodes generally connect between a patient and a physiological monitor monitoring a physiological condition of that patient, and the surface electrode provides the contact with the patient that enables measurement of potentials from the patient's body and conducts those potentials to the patient monitor. Thus, the surface electrodes are one of the key parts enabling physiological monitoring of biological signals, such as electrocardiograms (ECGs), electroencephalograms (EEGs), and respiration monitors. Surface electrodes generally include an electrode plate or section of conductive material that is electrically conductive with the patient's skin. The electrode plate is also galvanically connected to a leadwire that conducts potentials from the electrode plate to a patient monitor.

There are generally two categories, or types, of electrodes used in patient monitoring, including wet electrodes and dry electrodes. Dry electrodes consist of a metal that acts as a conductor-which may be a single metal such as silver or an alloy or some other metal-containing or conductive material-configured to connect to a leadwire. Wet electrodes are electrodes, typically made of silver/silver chloride material (Ag/ AgCl) or another metal, and configured to use an electrolytic gel material as a conductor between the skin and the electrode. Various concentrations and consistencies of electrolytic gel are utilized in different electrode applications.

US2008/0288026A1 describes an electro-mechanical connector for a thin medical monitoring patch. US2021/0345899A1 and WO2020/214812A1 describe multi-sensor patches.

### SUMMARY

This Summary is provided to introduce a selection of concepts that are further described below in the Detailed Description.

In one embodiment, a surface electrode for patient monitoring comprises a flexible substrate, a dry electrode on the substrate, and a wet electrode configured to contact an electrode gel in contact with a patient's skin. A conductive epoxy is arranged between the dry electrode and the wet electrode, and the conductive epoxy is configured to protect the dry electrode from corrosion and transfer electrical potentials from the wet electrode to the dry electrode. The conductive epoxy is deposited on a top surface of the dry electrode between the dry electrode and the wet electrode. The conductive epoxy is a continuous layer covering the entire top surface of the dry electrode.

In some embodiments of a surface electrode, conductive epoxy may be a silver-based epoxy. The conductive epoxy may be printed on the top surface of the dry electrode, and/or the conductive epoxy can be in contact with the wet electrode. Additionally or alternatively, the conductive epoxy may extend around the sides of the dry electrode.

In some embodiments of a surface electrode, the wet electrode may comprise a sponge impregnated with the electrode gel, and/or the electrode gel may be a sodium chloride gel or a potassium chloride gel. The wet gel electrode may comprise a foam ring defining an open central opening configured to receive the electrode gel, and an adhesive disposed on a top surface of the foam ring may be configured to secure the surface electrode to the patient's skin. Additionally or alternatively, the dry electrode may comprise silver, and/or the wet electrode may comprise silver/silver-chloride.

In one embodiment, a multi-electrode patch for detecting electrophysiological signals comprises a flexible substrate and a plurality of surface electrodes printed on the flexible substrate. Each of the plurality of surface electrodes comprise a printed dry electrode printed on the substrate, a wet electrode configured to contact an electrode gel in contact with a patient's skin, and a conductive epoxy. The conductive epoxy is arranged between the dry electrode and the wet electrode, and the conductive epoxy is configured to protect the printed dry electrode from corrosion and transfer electrical potentials from the wet electrode to the printed dry electrode. The conductive epoxy is deposited on a top surface of the dry electrode between the dry electrode and the wet electrode. The conductive epoxy is a continuous layer covering the entire top surface of the dry electrode.

In some embodiments of a multi-electrode patch, the conductive epoxy may comprise silver epoxy. The conductive epoxy may be deposited on a top surface of the dry electrode between the dry electrode and the wet electrode. Additionally or alternatively, the conductive epoxy of the plurality of surface electrodes may be printed on the printed dry electrodes of the plurality of surface electrodes.

In some embodiments of a multi-electrode patch, the conductive epoxy covers the entire top surface of the dry electrode and extends around the sides of the dry electrode. The printed dry electrode of each of the plurality of surface electrodes may be connected to a hub by a printed conductive track, and the conductive epoxy of each of the plurality of surface electrodes may include a spillover tab configured to protect a portion of the printed conductive track proximate the dry electrode of said surface electrode. Additionally or alternatively, the printed dry electrode may comprise silver and the wet electrode comprises silver/silver-chloride.

Various other features, objects, and advantages of the present disclosure will be made apparent from the following description taken together with the drawings.

### BRIEF DESCRIPTION OF THE DRAWINGS

The present disclosure is described with reference to the following Figures.
FIG. 1 depicts an embodiment of a multi-electrode patch.
FIG. 2 is a cross-sectional view of an embodiment of a surface electrode.
FIG. 3 is a cross-sectional view of another embodiment of a surface electrode.
FIG. 4 depicts an embodiment of a set of surface electrodes

### DETAILED DESCRIPTION

The present inventors have recognized several problems with currently available surface electrodes and electrode sets used in patient monitoring. Currently available surface electrodes, such as ECG electrodes, utilize an electrode gel to obtain the desired impedance between the surface electrode and a patient's skin and to ensure that physiological signals from the patient can be clearly detected and recorded. To obtain readings with an appropriate level of signal integrity, the electrode gel and a wet electrode must be comprised of materials having desired chemistry. For example, it may be important to use an electrode gel, also referred to as an electrolytic gel or conductive gel, with similar chloride salt content as the skin's surface to obtain reliable readings. Certain physiological monitoring applications where relatively high signal to noise ratio is desired may require the use of gel with higher electrolyte concentrations.

Some surface electrodes utilize a sodium chloride gel or a potassium chloride gel with a silver/silver chloride wet electrode. However, such electrodes have a limited shelf life due to the corrosiveness of the electrode gel on the electrode. The inventors have recognized that short shelf life is particularly a problem for printed electrodes, and the problem is most acute for printed wet electrodes configured to utilize electrode gels with relatively high sodium chloride or potassium chloride concentrations. For example, high concentration electrolytic gels are often used in maternal-fetal monitoring, where recording the relatively small amplitude fetal ECG signals requires a sufficiently high signal to noise ratio. Long term reliability testing performed by the inventors has identified a failure mode for printed wet electrodes where the electrode gel erodes and penetrates thru the silver/silver chloride to the silver electrode, eroding away the trace connection resulting in an open circuit.

Through research and experimentation in the relevant field, the present inventors have developed a surface electrode that uses a conductive epoxy between the dry electrode and the silver/silver chloride wet electrode to provide a barrier to the corrosive gels commonly used with wet electrodes. The conductive epoxy, such as a silver-based epoxy, is an interface between dry electrode pads connected to the signal traces and the silver/silver chloride of the wet electrode. In one embodiment, the conductive epoxy is screen printed over the dry electrode pads and positioned between dry electrode and the sliver/silver chloride wet electrode providing electrical connection with the electrode gel thru the wet electrode while also providing a barrier preventing the electrode gel from penetrating the dry electrode of conductive tracks.

The conductive epoxy is thus conductive of electrical potentials while also being resistant to corrosion by the electrode gel. For example, the conductive epoxy may be a silver-based epoxy. The conductivity epoxy could be any electrically conductive epoxy with a conductivity below 10E-3 ohm-cm, in some embodiments with a conductivity less than 10E-4 ohm-cm. The conductive epoxy may comprise carbon black and/or metal particles such as silver, copper, stainless steel, or mixture of these particles. Loading of these conductivity particles may be above 50% by weight, and in some embodiments may preferably be between 65-80% by weight. Particles size preferably will not exceed 50 microns, and in some embodiments may preferably be below 10 microns. Exemplary conductive epoxies include epoxy phenol novolac resin based fast curing epoxies.

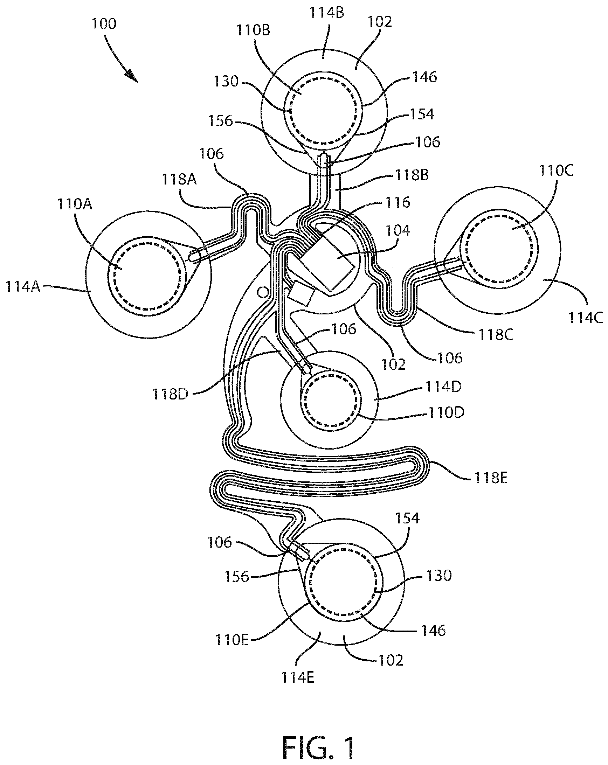

Referring to FIG. 1, an embodiment of a multi-electrode patch 100 used for maternal fetal monitoring is shown, viewed from the side that is to be facing the mother's abdomen in use. The multi-electrode patch 100 may include a flexible substrate 102 that supports a plurality of sensors, such as five surface electrodes 110A-E of the illustrated embodiment, a connection hub 104 for electrically connecting the surface electrodes 110 to a readout device (not shown), and conductive tracks 106 and/or leadwires electrically connecting the surface electrodes 110 to the connection hub 104. Some embodiments of a multi-electrode patch 100 may include a different number of surface electrodes 110. For example, a multi-electrode patch may include more than five surface electrodes or fewer than five surface electrodes. Additionally or alternatively, a multi-electrode patch 100 may include at least one surface electrode 110 that is in electrical communication with the readout device without the intermediate connection to the connection hub 104.

The flexible substrate 102 exemplarily defines the external shape of the multi-electrode patch 100 and includes sensor regions 114A-E exemplarily corresponding with each of the surface electrodes 110A-E. Each sensor region 114A-E may be linked to a central region 116 of the substrate 102 that supports the connection hub 104 by a connecting section 118A-E, which may be flexible or rigid. In the illustrated embodiment, for example, two sensor regions 114B, 114D are connected to the central region 116 by rigid connecting sections 118B, 118D while flexible connecting sections 118A,118C,118E link the remaining three sensor regions 114A,114C,114E to the central region 116. Each of the flexible connecting sections 118A,118C,118E is configured to be deformable to allow the relative positions of the corresponding sensor regions 114A,114C,114E to be adjusted when the substrate 102 is conformed to a surface (such as an abdomen), thereby altering the positions of the surface electrodes 110A,110C,110E, relative at least one of each other, the other surface electrodes 110B, 110E, and the central region 116 of the patch 100.

With continued reference to FIG. 1, at least one of the surface electrodes 110, the conductive tracks 106, and any auxiliary sensors or other electronics may be at least partially formed in a signal layer printed or otherwise disposed on the substrate 102. For example, in the illustrated embodiments one or more of the surface electrodes 110A-E and the conductive tracks 106 may be printed on the substrate 102 with a conductive ink, such as a silver-based ink, a gold-based ink, a copper-based ink, and any other type of conductive ink. Additionally or alternatively, some embodiments may include at least one surface electrode 110 that is connected to the connection hub 104 via a leadwire and/or a wireless connection.

To protect the conductive tracks 106 from external interference, embodiments of a multi-electrode patch 100 may include a substrate 102 with at least one layer of electrical insulation. For example, a substrate 102 may include a polymer base layer on which the signal layer is formed and at least one insulating layer formed between the base layer of the substrate 102 and the signal layer. Insulating layers may comprise at least one of an insulating dielectric layer, a graphite layer, a conductive shield layer, and any other type of layer configured to electrically isolate the signal layer from the environment. The substrate 102 may further include an overlaminate layer formed above and below the base layer, the signal layer, and any insulating layers such that they are substantially encapsulated by the overlaminate. In such an embodiment, the overlaminate and insulating layers may be omitted proximate the sensor regions 114 so that the connection hub 104 and/or the surface electrodes 110 are exposed. For example, in the illustrated embodiments the signal layer is exposed in the area about the surface electrodes 110 so that they can make contact with an underlying surface of the maternal patient's skin. It should be appreciated that embodiments of the substrate may include a variety of different layers layered in various different orders. Some embodiments of a substrate may comprise a single layer of material.

For any polymer layer of the substrate 102 described above, such as the base layer and/or the overlaminate layer, a PET material may be used and has been found to provide useful properties, e.g., resilience, for avoiding breakage of the signal layer during flexing of the patch in use. Alternatively, other polymer materials may be used as the flexible substrate, such as Kapton or other polyimides, or a thermoplastic polyurethane (TPU). The material thickness of the flexible substrate layer(s) may be matched to the properties of the conductive tracks 106 to prevent deformation of the conductive tracks 106 in a manner that may lead to a break in the signal layer. Additionally or alternatively, embodiments of a multi-electrode patch 100 may include a substrate 102 with a portion of the base layer and/or the overlaminate that is formed from a type of material other than polymer.

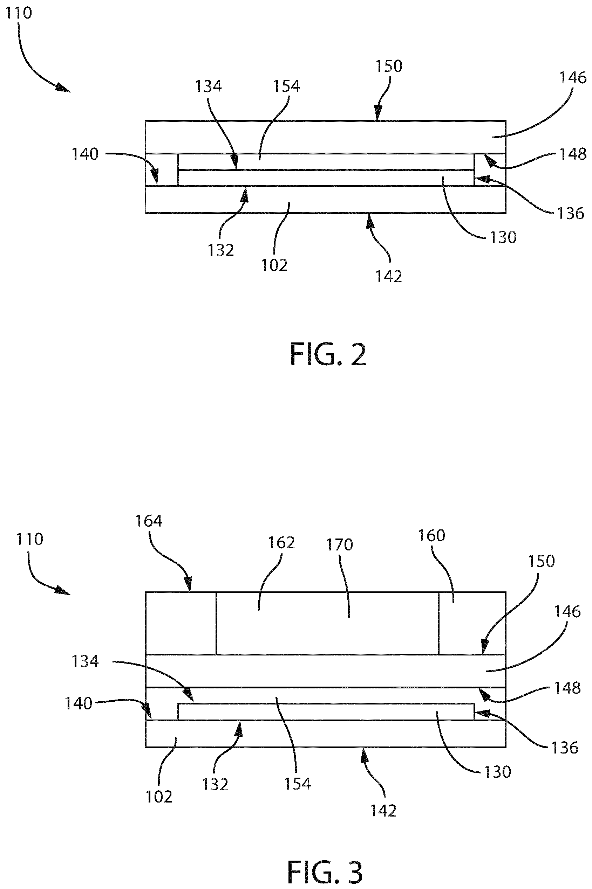

As previously mentioned, a surface electrode 110 may be positioned at each of the sensor regions 114 of the multi-electrode patch 100. The surface electrodes 110 may be disposed on the substrate 102 such that a portion of the surface electrode 110 is in electrical communication with a corresponding conductive track 106. Looking to FIGS. 2 and 3, a cross-sectional view of embodiments of a surface electrode 110 are shown. The surface electrodes 110 may include a plurality of layers that are printed onto the substrate 102. It should be appreciated that, while the substrate 102 is depicted as having a single layer in FIGS. 2 and 3, the substrate may be formed by multiple layers as described above.

In the illustrated embodiments, each surface electrode 110 includes a dry electrode 130 positioned above the substrate 102 such that the bottom surface 132 of the dry electrode layer 130 is adjacent to a top side 140 of the substrate 102 opposite a bottom side 142 of the substrate 102. The dry electrode 130 may be a metal-containing plate, such as a printed plate or a thin metal plate or disk comprising silver, copper, nickel-silver, and or another suitable metal or metal alloy. In some embodiments the dry electrode 130 may be a metal disc, plate, etc. adhered or otherwise applied to the substrate, rather than printed. The dry electrode layer 130 may be a flexible conductive or rigid conductive element, such as a printed dry electrode layer 130 comprised of a conductive ink, such as a silver-based ink. In such an embodiment, the dry electrode layer 130 may be printed as part of the conductive track 106, or it may be printed onto the sensor region 114 of the substrate 102 in a separate layer.

Each surface electrode 110 may further include a wet electrode 146 configured to contact an electrode gel (electrode gel 170 shown in FIG. 3) that is in contact with a patient's skin. In the illustrated embodiments, the wet electrode 146 is positioned above the dry electrode 130 and may comprise silver/silver chloride due to silver/silver chloride's compatibility with the chloride-based electrode gels. However, some embodiments of a surface electrode 110 may include a wet electrode 146 comprised of a different material other than silver/silver chloride, such as gold or copper.

The electrode gel may be disposed on the top surface 150 of the wet electrode 146. In the embodiment of FIG. 2, the electrode gel may be applied directly to the top surface 150 of the wet electrode 146 immediately prior to use of the electrode on a patient, such as applied by a clinician at the time of placing the surface electrode 110 on the patient. Other embodiments may include a structure for retaining the electrode gel on the wet electrode 146. For example, as illustrated in FIG. 3, a foam ring 160 may be positioned on the top surface 150. The foam ring 160 may extend around the periphery of the wet electrode 146 and defines a central opening 162 that is configured to receive the electrode gel 170 and retain it therein. In some embodiments, the electrode gel 170 may be directly inserted into the central opening 162 of the foam ring 160, such as by a clinician at the time of use. Additionally or alternatively, the central opening 162 may be configured to receive a sponge that is impregnated with the electrode gel 170, which in some embodiments is assembled onto the surface electrode 110 at the time of manufacture. Other embodiments may use a different structure or method for retaining the electrode gel 170 on the wet electrode 146. For example, a sponge impregnated with the electrode gel 170 may be positioned on the top surface 150 of the wet electrode 146 without the use of a foam ring.

With continued reference to FIGS. 2 and 3, the wet electrode 146 is spaced apart from the dry electrode 130 by a conductive epoxy 154. The conductive epoxy 154 is disposed directly on a top surface 134 of the dry electrode 130 and adjacent a bottom surface 148 of the wet electrode 146. The conductive epoxy layer 154 is configured to transfer electrical potentials from the wet electrode 146 to the dry electrode 130 while also acting as a barrier to protect the dry electrode 130 from possible corrosion by the electrode gel. The conductive epoxy 154 may be deposited between the dry electrode 130 and the wet electrode 146 through a variety of different methods and in a variety of different configurations and patterns. For example, in the embodiment of FIG. 2, the conductive epoxy 154 is printed across the entire top surface 134 of the dry electrode and is in direct contact with the bottom surface 148 of the wet electrode 146. The space 168 adjacent the sides 136 of the dry electrode 130 may be left open or it may be filled with another material, such as a non-conductive epoxy, a polymer, and/or another type of insulating material. In the embodiment of FIG. 3, the conductive epoxy 154 may extend around the sides 136 of the dry electrode 130 to fill the space adjacent the dry electrode 130 and partially or fully encapsulate the dry electrode 130. This may be useful, for example, to protect the sides 136 of the dry electrode.

In the illustrated embodiments, the conductive epoxy 154 is printed on the dry electrode 130 in a continuous layer that covers the entire top surface 134 of the dry electrode 130.

The conductive epoxy 154 of the illustrated embodiments may be a silver-based epoxy. As discovered by the inventors through extensive experimentation, a silver-based epoxy may be particularly resistant to corrosion by the electrode gel while still possessing the conductivity required to pass electrical potentials from the wet electrode 146 to the dry electrode 130 without compromising the signal integrity. This may be useful, for example, to extend the shelf life of the surface electrodes 110 and/or to enable prolonged use of the surface electrode 110 on a patient for monitoring. Other embodiments, however, may include a different type of conductive epoxy.

As illustrated in FIG. 1, the conductive epoxy layer 154 may include a spillover tab 156 that projects radially outward from the side of the surface electrode 110 to cover a portion of the conductive track 106 that is connected to the dry electrode 130. This may be useful, for example, to protect the conductive track 106 (and any other covered portion of the signal layer) from corrosion by electrode gel that spills over from the wet electrode 146. Some embodiments, however, may omit the spillover tab 156.

Some embodiments of a surface electrode 110 may include a conductive epoxy 154 arranged in a different configuration. For example, a conductive epoxy 154 may be deposited on the dry electrode 130 through a process other than screen printing, and/or the conductive epoxy 154 may be arranged in a different pattern on the top surface 134 of the dry electrode 130 than the patterns of the illustrated embodiments. In the illustrated embodiments, the conductive epoxy layer 154 is in direct contact with the dry electrode 130 and the wet electrode. In some embodiments, however, a surface electrode 110 may include at least one additional layer of material between the dry electrode 130 and the wet electrode 146 so that the conductive epoxy 154 is not in contact with the wet electrode 146.While the illustrated dry electrodes 130 and the wet electrodes 146 are depicted as substantially circular, it should be recognized that the sensors may be arranged in any shape as is suitable for the measurement obtained by the surface electrodes.

Returning to FIG. 1, the sensor regions 114 of the substrate 102 may be provided with an adhesive film around the perimeter of the surface electrodes 110, so that each sensor region 114 can be adhered to the skin of a subject. Additionally or alternatively, embodiments of the multi-electrode patch 100 that include surface electrodes 110 that include a foam ring 160, an adhesive may be disposed on a top surface 164 of the foam ring 160 so that the surface electrode is held against the patient's skin. In some embodiments, at least one sensor region 114 of the substrate 102 may include a lobe or flap (not shown) that is substantially free from adhesive film or conducting medium and protrudes from the patch region sensor region 114. The lobe may be used by a clinician to hold the respective sensor region 114 for placement, movement, and/or detachment of the sensor region 114 on the maternal patient's body. Clinician gripping of the lobe may help to prevent fouling or contamination of any adhesive or conducting medium of the sensor region 114 and/or cross-contamination to the clinician from handling a sensor region 114 that has been used.

While the multi-electrode patch 100 includes multiple sensor regions 114, connecting sections 118, and other regions of the substrate 102 that are separate from each other, at least some of the features of these separate regions may be formed in a single process. For example, at least one of the dry electrodes 130, the conductive epoxy 154, and the wet electrodes 146 of two or more of the individual surface electrodes 110 may be formed in a single printing process. In some embodiments, at least one of the conductive tracks 106, the dry electrodes 130, the conductive epoxy 154, and the wet electrodes 146 may be printed in a single layer across the substrate 102. Additionally or alternatively, a portion of at least one of the surface electrodes 110 may be individually formed in a separate process. Further still, some embodiments of a multi-electrode patch 100 may include at least one modular surface electrode 110 that is separately manufactured and inserted onto the patch 100.

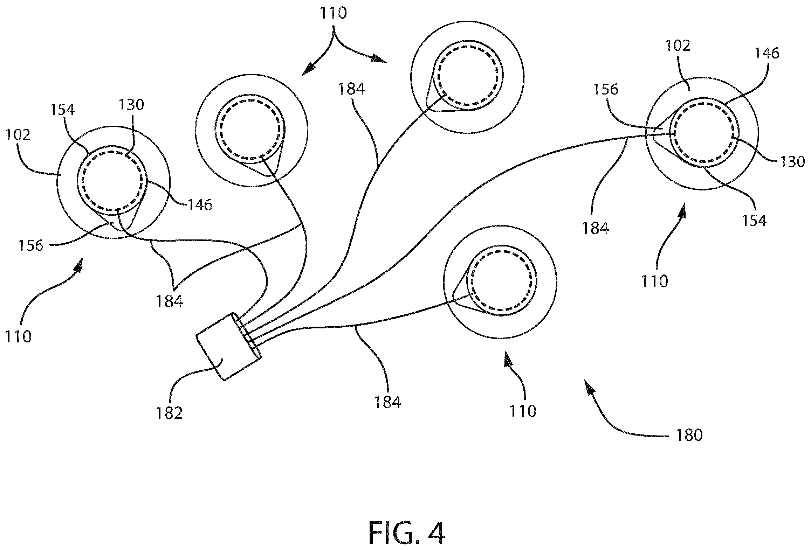

Some embodiments of a surface electrode may be configured for individual use without a multi-electrode patch. For example, FIG. 4 illustrates an embodiment of a set 180 of individual surface electrodes 110. As illustrated in FIGS. 2 and 3, each of the surface electrodes 110 may include a substrate 102, a dry electrode 130 printed or otherwise disposed onto the substrate 102, a wet electrode 146 configured to contact an electrode gel in contact with a patient's skin, and a conductive epoxy layer 154 configured to protect the dry electrode 130 from corrosion and transfer electrical potentials from the wet electrode 146 to the dry electrode 130. The dry electrode 130 of each surface electrode 110 may be electrically connected, via a leadwire 184, to a connection hub 182 configured to connect to a patient monitor, thereby providing an electrical connection between each surface electrode 110 and the patient monitor. Similarly to the embodiment of FIG. 1, the conductive epoxy layer 154 of each surface electrode 110 may include a spillover tab 156 configured to protect the connection between the dry electrode 130 and the leadwire 184.

Embodiments of a set 180 of surface electrodes 110 may include any number of one or more surface electrodes 110. In the example at FIG. 4, the set 180 of surface electrodes 110 includes five surface electrodes 110. Other embodiments of a set of surface electrodes 110 may include more than five surface electrodes 110 or fewer than five surface electrodes 110. In some embodiments, at least one of the surface electrodes may be different than another surface electrode. Other embodiments of a set of surface electrodes may include a plurality of substantially identical surface electrodes. In still other embodiments, a single surface electrode 110 having the features disclosed herein may be utilized.

Certain terms have been used for brevity, clarity and understanding. No unnecessary limitations are to be inferred therefrom beyond the requirement of the prior art because such terms are used for descriptive purposes only and are intended to be broadly construed. The scope of the invention is defined by the claims.

Unless otherwise specified or limited, the terms "mounted," "connected," "linked," "supported," and "coupled" and variations thereof are used broadly and encompass both direct and indirect mountings, connections, supports, and couplings. Further, unless otherwise specified or limited, "connected" and "coupled" are not restricted to physical or mechanical connections or couplings. As used herein, unless otherwise limited or defined, discussion of particular directions is provided by example only, with regard to particular embodiments or relevant illustrations. For example, discussion of "top," "bottom," "front," "back," "left" or "right" features is generally intended as a description only of the orientation of such features relative to a reference frame of a particular example or illustration. Correspondingly, for example, a "top" feature may sometimes be disposed below a "bottom" feature (and so on), depending on the position of the element and the frame of reference. Additionally, use of the words "first," "second", "third," etc. is not intended to connote priority or importance, but merely to distinguish one of several similar elements or machines from another.

## Claims

1. A surface electrode (110) for patient monitoring comprising:
a flexible substrate (102);
a dry electrode (130) on the substrate;
a wet electrode (146) configured to contact an electrode gel in contact with a patient's skin;
a conductive epoxy (154) arranged between the dry electrode and the wet electrode, the conductive epoxy configured to protect the dry electrode from corrosion and to transfer electrical potentials from the wet electrode to the dry electrode, wherein the conductive epoxy is deposited on a top surface (134) of the dry electrode between the dry electrode and the wet electrode and wherein the conductive epoxy is a continuous layer covering the entire top surface of the dry electrode.

2. The surface electrode of claim 1, wherein the conductive epoxy (154) is in direct contact with the wet electrode (146).

3. The surface electrode of claim 1, wherein the conductive epoxy is a silver-based epoxy.

4. The surface electrode of claim 1, wherein the conductive epoxy extends around the sides (136) of the dry electrode.

5. The surface electrode of claim 1, wherein the conductive epoxy is printed on the top surface of the dry electrode.

6. The surface electrode of claim 1, wherein the conductive epoxy is in contact with the wet electrode.

7. The surface electrode of claim 1, wherein the conductive epoxy is printed over a top surface (134) of the dry electrode.

8. The surface electrode of claim 1, wherein the wet electrode comprises a sponge impregnated with the electrode gel.

9. The surface electrode of claim 1, wherein the electrode gel is a sodium chloride gel or a potassium chloride gel.

10. The surface electrode of claim 1, wherein the dry electrode comprises silver.

11. The surface electrode of claim 1, wherein the wet electrode comprises silver/silver-chloride.

12. A multi-electrode patch (100) for detecting electrophysiological signals, the multi-electrode patch comprising:
a flexible substrate (102);
a plurality of surface electrodes (110) printed on the flexible substrate, each of the plurality of surface electrodes comprising:
a printed dry electrode (130) printed on the substrate;
a wet electrode (146) configured to contact an electrode gel in contact with a patient's skin; and
a conductive epoxy (154) arranged between the printed dry electrode and the wet electrode, the conductive epoxy configured to protect the printed dry electrode from corrosion and transfer electrical potentials from the wet electrode to the printed dry electrode, wherein the conductive epoxy is deposited on a top surface (134) of the dry electrode between the dry electrode and the wet electrode and wherein the conductive epoxy is a continuous layer covering the entire top surface of the dry electrode.

13. The multi-electrode patch of claim 12, wherein the conductive epoxy of the plurality of surface electrodes is printed on the printed dry electrodes of the plurality of surface electrodes.

14. The multi-electrode patch of claim 12, wherein the printed dry electrode of each of the plurality of surface electrodes is connected to a hub (104) by a printed conductive track, and wherein the conductive epoxy of each of the plurality of surface electrodes includes a spillover tab configured to protect a portion of the printed conductive track proximate the dry electrode of said surface electrode.

## Patentansprüche

1. Oberflächenelektrode (110) zur Patientenüberwachung, umfassend:
ein flexibles Substrat (102);
eine Trockenelektrode (130) auf dem Substrat;
eine Nasselektrode (146), ausgelegt zum Kontakt mit einem Elektrodengel in Kontakt mit der Haut eines Patienten;
ein leitfähiges Epoxid (154), angeordnet zwischen der Trockenelektrode und der Nasselektrode, wobei das leitfähige Epoxid dazu ausgelegt ist, die Trockenelektrode vor Korrosion zu schützen und elektrische Potentiale von der Nasselektrode auf die Trockenelektrode zu übertragen, wobei das leitfähige Epoxid auf einer oberen Oberfläche (134) der Trockenelektrode zwischen der Trockenelektrode und der Nasselektrode abgeschieden ist und wobei das leitfähige Epoxid eine kontinuierliche Schicht ist, die die gesamte obere Oberfläche der Trockenelektrode bedeckt.

2. Oberflächenelektrode nach Anspruch 1, wobei das leitfähige Epoxid (154) in direktem Kontakt mit der Nasselektrode (146) vorliegt.

3. Oberflächenelektrode nach Anspruch 1, wobei das leitfähige Epoxid ein Epoxid auf Silberbasis ist.

4. Oberflächenelektrode nach Anspruch 1, wobei das leitfähige Epoxid um die Seiten (136) der Trockenelektrode verläuft.

5. Oberflächenelektrode nach Anspruch 1, wobei das leitfähige Epoxid auf die obere Oberfläche der Trockenelektrode gedruckt ist.

6. Oberflächenelektrode nach Anspruch 1, wobei das leitfähige Epoxid in Kontakt mit der Nasselektrode vorliegt.

7. Oberflächenelektrode nach Anspruch 1, wobei das leitfähige Epoxid über eine obere Oberfläche (134) der Trockenelektrode gedruckt ist.

8. Oberflächenelektrode nach Anspruch 1, wobei die Nasselektrode einen mit dem Elektrodengel imprägnierten Schwamm umfasst.

9. Oberflächenelektrode nach Anspruch 1, wobei das Elektrodengel ein Natriumchloridgel oder ein Kaliumchloridgel ist.

10. Oberflächenelektrode nach Anspruch 1, wobei die Trockenelektrode Silber umfasst.

11. Oberflächenelektrode nach Anspruch 1, wobei die Nasselektrode Silber/Silberchlorid umfasst.

12. Mehrelektrodenpatch (100) zum Erfassen elektrophysiologischer Signale, wobei das Mehrelektrodenpatch umfasst:
ein flexibles Substrat (102);
mehrere Oberflächenelektroden (110), die auf das flexible Substrat gedruckt sind, wobei jede der mehreren Oberflächenelektroden umfasst:
eine gedruckte Trockenelektrode (130), die auf das Substrat gedruckt ist;
eine Nasselektrode (146), ausgelegt zum Kontakt mit einem Elektrodengel in Kontakt mit der Haut eines Patienten;
und
ein leitfähiges Epoxid (154), angeordnet zwischen der gedruckten Trockenelektrode und der Nasselektrode, wobei das leitfähige Epoxid dazu ausgelegt ist, die gedruckte Trockenelektrode vor Korrosion zu schützen und elektrische Potentiale von der Nasselektrode auf die gedruckte Trockenelektrode zu übertragen, wobei das leitfähige Epoxid auf einer oberen Oberfläche (134) der Trockenelektrode zwischen der Trockenelektrode und der Nasselektrode abgeschieden ist und wobei das leitfähige Epoxid eine kontinuierliche Schicht ist, die die gesamte obere Oberfläche der Trockenelektrode bedeckt.

13. Mehrelektrodenpatch nach Anspruch 12, wobei das leitfähige Epoxid der mehreren Oberflächenelektroden auf die gedruckten Trockenelektroden der mehreren Oberflächenelektroden gedruckt ist.

14. Mehrelektrodenpatch nach Anspruch 12, wobei die gedruckte Trockenelektrode jeder der mehreren Oberflächenelektroden durch eine gedruckte leitfähige Bahn mit einem Knotenpunkt (104) verbunden ist und wobei das leitfähige Epoxid jeder der mehreren Oberflächenelektroden eine Überlauflasche aufweist, die dazu ausgelegt ist, einen Teil der gedruckten leitfähigen Bahn nahe der Trockenelektrode der Oberflächenelektrode zu schützen.

## Revendications

1. Électrode de surface (110) destinée à la surveillance d'un patient et comprenant :
un substrat souple (102) ;
une électrode sèche (130) sur le substrat ;
une électrode humide (146) configurée pour mettre en contact un gel d'électrode en contact avec la peau d'un patient ;
un époxy conducteur (154) agencé entre l'électrode sèche et l'électrode humide, l'époxy conducteur étant configuré pour protéger l'électrode sèche contre la corrosion et pour transférer des potentiels électriques de l'électrode humide à l'électrode sèche, l'époxy conducteur étant déposé sur une surface supérieure (134) de l'électrode sèche entre l'électrode sèche et l'électrode humide, et l'époxy conducteur étant une couche continue recouvrant toute la surface supérieure de l'électrode sèche.

2. Électrode de surface selon la revendication 1, l'époxy conducteur (154) étant en contact direct avec l'électrode humide (146).

3. Électrode de surface selon la revendication 1, l'époxy conducteur étant un époxy à base d'argent.

4. Électrode de surface selon la revendication 1, l'époxy conducteur s'étendant autour des côtés (136) de l'électrode sèche.

5. Électrode de surface selon la revendication 1, l'époxy conducteur étant imprimé sur la surface supérieure de l'électrode sèche.

6. Électrode de surface selon la revendication 1, l'époxy conducteur étant en contact avec l'électrode humide.

7. Électrode de surface selon la revendication 1, l'époxy conducteur étant imprimé sur une surface supérieure (134) de l'électrode sèche.

8. Électrode de surface selon la revendication 1, l'électrode humide comprenant une éponge imprégnée du gel d'électrode.

9. Électrode de surface selon la revendication 1, le gel d'électrode étant un gel de chlorure de sodium ou un gel de chlorure de potassium.

10. Électrode de surface selon la revendication 1, l'électrode sèche comprenant de l'argent.

11. Électrode de surface selon la revendication 1, l'électrode humide comprenant de l'argent/chlorure d'argent.

12. Patch multi-électrodes (100) de détection de signaux électrophysiologiques, le patch multi-électrodes comprenant :
un substrat souple (102) ;
une pluralité d'électrodes de surface (110) imprimées sur le substrat souple, chacune parmi la pluralité d'électrodes de surface comprenant :
une électrode sèche imprimée (130) imprimée sur le substrat ;
une électrode humide (146) configurée pour mettre en contact un gel d'électrode en contact avec la peau d'un patient ; et
un époxy conducteur (154) agencé entre l'électrode sèche imprimée et l'électrode humide, l'époxy conducteur étant configuré pour protéger l'électrode sèche imprimée contre la corrosion et transférer les potentiels électriques de l'électrode humide à l'électrode sèche imprimée, l'époxy conducteur étant déposé sur une surface supérieure (134) de l'électrode sèche entre l'électrode sèche et l'électrode humide, et l'époxy conducteur étant une couche continue recouvrant toute la surface supérieure de l'électrode sèche.

13. Patch à électrodes multiples selon la revendication 12, l'époxy conducteur de la pluralité d'électrodes de surface étant imprimé sur les électrodes sèches imprimées de la pluralité d'électrodes de surface.

14. Patch à électrodes multiples selon la revendication 12, l'électrode sèche imprimée de chacune de la pluralité d'électrodes de surface étant connectée à un hub (104) par une piste conductrice imprimée, et l'époxy conducteur de chacune de la pluralité d'électrodes de surface comprenant une languette de débordement configurée pour protéger une partie de la piste conductrice imprimée à proximité de l'électrode sèche de ladite électrode de surface.
